# EUROPÄISCHE PATENTSCHRIFT

(11) **EP 3 379 487 B1**
(45) Veröffentlichungstag und Bekanntmachung des Hinweises auf die Patenterteilung: **04.10.2023**
(21) Anmeldenummer: 17162007.3
(22) Anmeldetag: 21.03.2017
(51) Int. Cl.: G06T 5/00, G06T 5/50

(54) **KONTRASTVERSTÄRKTE WIEDERGABE VON SPEKTRALEN CT-BILDDATEN**
CONTRAST ENHANCED REPRODUCTION OF SPECTRAL CT IMAGE DATA
REPRODUCTION À CONTRASTE RENFORCÉ DE DONNÉES D'IMAGERIE SCANNER SPECTRALE

(43) Veröffentlichungstag der Anmeldung: 26.09.2018
(73) Patentinhaber: Siemens Healthcare GmbH, 91052 Erlangen (DE)
(72) Erfinder: Lichy, Matthias, 90489 Nürnberg (DE); Schmidt, Sebastian, 91085 Weisendorf (DE)

(56) Entgegenhaltungen:
- US-A1- 2005 163 283
- US-A1- 2006 109 949
- RAJBHANDARY PAURAKH L ET AL: ""Conventional" CT images from spectral measurements", PROGRESS IN BIOMEDICAL OPTICS AND IMAGING, SPIE - INTERNATIONAL SOCIETY FOR OPTICAL ENGINEERING, BELLINGHAM, WA, US, Bd. 9783, 29. März 2016 (2016-03-29), Seiten 97831Q-97831Q, XP060066098, ISSN: 1605-7422, DOI: 10.1117/12.2216988 ISBN: 978-1-5106-0027-0
- SCHMIDT TALY: "Optimal image-based weighting for energy-resolved CT", MEDICAL PHYSICS, AIP, MELVILLE, NY, US, Bd. 36, Nr. 7, 10. Juni 2009 (2009-06-10), Seiten 3018-3027, XP012130093, ISSN: 0094-2405, DOI: 10.1118/1.3148535
- CRAMER TODD W ET AL: "A primer on the use of dual-energy CT in the evaluation of commonly encountered neoplasms", ABDOMINAL RADIOLOGY, SPRINGER US, NEW YORK, Bd. 41, Nr. 8, 30. März 2016 (2016-03-30), Seiten 1618-1631, XP036021565, ISSN: 2366-004X, DOI: 10.1007/S00261-016-0707-X [gefunden am 2016-03-30]
- B. Glocker ET AL: "Random Forests for Localization of Spinal Anatomy" In: "Medical Image Recognition, Segmentation and Parsing", 2. Dezember 2015 (2015-12-02), Elsevier, XP055391982, ISBN: 978-0-12-802581-9 Seiten 93-110, DOI: 10.1016/B978-0-12-802581-9.00005-6, * Seite 94, Absatz 1 *
- MERTENS T ET AL: "Exposure Fusion", COMPUTER GRAPHICS AND APPLICATIONS, 2007. PG '07. 15TH PACIFIC CONFERENCE ON, IEEE, PISCATAWAY, NJ, USA, 29. Oktober 2007 (2007-10-29), Seiten 382-390, XP031338475, ISBN: 978-0-7695-3009-3
- LIFENG YU ET AL: "Dual-Energy CT-Based Monochromatic Imaging", AMERICAN JOURNAL OF ROENTGENOLOGY, Bd. 199, Nr. 5_supplement, 1. November 2012 (2012-11-01), Seiten S9-S15, XP055392243, US ISSN: 0361-803X, DOI: 10.2214/AJR.12.9121

## Beschreibung

Die Erfindung betrifft ein Verfahren zur Einstellung eines Kontrasts einer Multi-Energie-CT-Bilddarstellung. Zudem betrifft die Erfindung ein Multi-Energie-CT-Bildgebungsverfahren. Weiterhin betrifft die Erfindung eine Einstelleinrichtung. Überdies betrifft die Erfindung ein Computertomographiesystem.

Mit Hilfe moderner bildgebender Verfahren werden häufig zwei- oder dreidimensionale Bilddaten erzeugt, die zur Visualisierung eines abgebildeten Untersuchungsobjekts und darüber hinaus auch für weitere Anwendungen genutzt werden können.

Häufig basieren die bildgebenden Verfahren auf der Erfassung von Röntgenstrahlung, wobei sogenannte Projektionsmessdaten erzeugt werden. Beispielsweise können Projektionsmessdaten mit Hilfe eines Computertomographiesystems (CT-Systems) akquiriert werden. Bei CT-Systemen läuft gewöhnlich eine an einer Gantry angeordnete Kombination aus Röntgenquelle und gegenüberliegend angeordnetem Röntgendetektor um einen Messraum um, in dem sich das Untersuchungsobjekt (das im Folgenden ohne Beschränkung der Allgemeinheit als Patient bezeichnet wird) befindet. Das Drehzentrum (auch "Isozentrum" genannt) fällt dabei mit einer sogenannten Systemachse z zusammen. Bei einem oder mehreren Umläufen wird der Patient mit Röntgenstrahlung der Röntgenquelle durchstrahlt, wobei mit Hilfe des gegenüberliegenden Röntgendetektors Projektionsmessdaten bzw. Röntgenprojektionsdaten erfasst werden.

Die erzeugten Projektionsmessdaten sind insbesondere von der Bauart des Röntgendetektors abhängig. Röntgendetektoren weisen gewöhnlich eine Mehrzahl an Detektionseinheiten auf, die meist in Form eines regelmäßigen Pixelarrays angeordnet sind. Die Detektionseinheiten erzeugen jeweils für auf die Detektionseinheiten auftreffende Röntgenstrahlung ein Detektionssignal, welches zu bestimmten Zeitpunkten hinsichtlich Intensität und spektraler Verteilung der Röntgenstrahlung analysiert wird, um Rückschlüsse auf das Untersuchungsobjekt zu erhalten und Projektionsmessdaten zu erzeugen.

Die ermittelten Intensitäten entsprechen sogenannten Schwächungswerten, die in sogenannten Hounsfield-Einheiten (abgekürzt HU = Hounsfield Unit) ausgedrückt werden. Zahlenwerte der Schwächungswerte liegen zwischen -1000 und einigen tausend im positiven Bereich. Diesen HU-Werten werden in der CT-Bildgebung Graustufen zugeordnet. Da der Mensch nicht in der Lage ist, derart viele Graustufen zu unterscheiden, wurde das Verfahren der "Fensterung" eingeführt, bei dem ein einstellbarer Teil der HU-Skala linear auf Graustufen von Weiß bis Schwarz abgebildet wird. Bestimmte Parameter der Fensterung kann der Benutzer frei einstellen, wobei das Zentrum (im Englischen "Center") und die Breite des Fensters (im Englischen "Width") in HU angegeben werden. Alle HU-Werte innerhalb des Fensters werden dann auf die verfügbaren Graustufen abgebildet, von denen das menschliche Auge ungefähr 60 Werte unterscheiden kann. Die Wahl der HU-Werte für Center und Width werden dabei an den jeweiligen Untersuchungsbereich und Untersuchungszweck angepasst. Beispielsweise hängt die Wahl der HU-Werte von den zu untersuchenden Organen und den damit verbundenen klinisch relevanten Dichtewerten ab. Diese klinisch als "Fenster" eingestellten Werte unterscheiden sich je nach Anwendungsbereich erheblich.

Eine moderne Form der CT-Bildgebung ist die sogenannte spektrale CT-Bildgebung. Hierbei werden Röntgenstrahlen nach Röntgenenergie aufgelöst erfasst. Da Photonen unterschiedlicher Energie von verschiedenen Materialien unterschiedlich absorbiert werden, können mit einem solchen Bildgebungsverfahren unterschiedliche Materialien getrennt erfasst werden. Ein Beispiel hierfür ist die Darstellung der Verteilung von in Gefäßen aufgenommenem Kontrastmittel. Für eine solche Darstellung werden sogenannte Falschfarbendarstellungen generiert.

Bei vier oder mehr aufgelösten Photonenenergien kommt es zu Bilddarstellungen, bei denen je nach Gewichtung der Energiebänder ganz unterschiedliche Bilder erzeugt werden. Im Vergleich zur Fensterung besteht bei der Gewichtung der einzelnen Energiebänder anstatt eines linearen eindimensionalen Problems bei der Umrechnung von Graustufen ein multidimensionales Problem. Mithin ist es für einen Benutzer sehr schwer oder gar kaum noch möglich, einen für seine Zwecke idealen Kontrast einzustellen.

Bei der photographischen Bildgebung gibt es Verfahren zur Kontrastwahl, bei denen bestimmte Teile des Farbspektrums mit Hilfe eines Farbfilters absorbiert werden, um Kontraste in anderen Teilen des Spektrums hervorzuheben. Beispielsweise wird in der Schwarzweißfotographie zur Darstellung von Wolken ein Gelbfilter genutzt, damit der Himmel dunkler erscheint und die weißen Wolken stärker hervortreten. Eine solche Kontrastwahl funktioniert jedoch bereits bei dem Dreifarbensystem des menschlichen Sehens nur bei wenigen Anwendungen.

In der Endoskopie wird eine Bildgebung mit Hilfe eines sogenannten "Narrow Band imaging" erzielt. Ein solches Verfahren ist in https://www.olympus.de/medical/de/medical_systems /applications/gastroenterology_1/narrow_band_imaging_nbi_in _gastroenterology/narrow_band_imaging_nbi_2.jsp beschrieben. Dabei werden bestimmte Energiebänder ausgefiltert, so dass die verbleibenden Energieanteile einen maximalen Kontrast für bestimmte Strukturen, beispielsweise Blutgefäße gegenüber Schleimhaut, ergeben. Eine solche Vorgehensweise funktioniert in der Gastroenterologie gut, weil stets die gleichen Kontraste benötigt werden.

Für die Radiologie mit ihrer viel größeren Variabilität von darzustellenden Strukturen, beispielsweise Läsionen, ist ein derartiger "Einheitsfilter" jedoch nicht geeignet.

In Paurakh L et al: "Conventional CT images from spectral measurements", PROGRESS IN BIOMEDICAL OPTICS AND IMAGING, SPIE-INTERNATIONAL SOCIETY FOR OPTICAL ENGINEERING, BELLINGHAM, WA, US, Bd. 9783, 29. März (2016-03-29), Seiten 97831Q-97831Q, XP060066098, ISSN: 1605-7422, DOI: 10.1117/12.2216988 ISBN: 978-1-5106-0027-0 wird eine Gewichtung von Spektralbildern zur Kompensation der Röntgenstrahlaufhärtung bei der CT-Bildgebung beschrieben.

In Schmidt TALY: "Optimal image-based weighting for energyresolved CT", MEDICAL PHYSICS, AIP, MELVILLE, NY, US, Bd. 36, Nr. 7, 10. Juni 2009 (2009-06-10) wird eine bildbasierte Gewichtung im Zusammenhang mit der multispektralen CT-Bildgebung beschrieben, wobei die Gewichte proportional zu einem um eine Varianz genormten Kontrastwert gewählt werden.

In US 2006/ 0 109 949 A1 wird eine Gewichtung von spektralen Bildern einer Multi-Energie-CT-Bildgebung auf Basis einer Materialanalyse in einem Bildbereich beschrieben.

Es besteht also das Problem, ein Verfahren und eine Anordnung zum Einstellen eines geeigneten Kontrasts für die Darstellung von gewichteten Bildern auf Basis einer Multi-Energie-CT-Bildgebung anzugeben.

Diese Aufgabe wird durch ein Verfahren zur Einstellung eines Kontrasts einer Multi-Energie-CT-Bilddarstellung gemäß Patentanspruch 1, ein Multi-Energie-CT-Bildgebungsverfahren gemäß Patentanspruch 8, eine Bildeinstelleinrichtung gemäß Patentanspruch 9, ein Computertomographiesystem gemäß Patentanspruch 10, ein Computerprogrammprodukt gemäß Anspruch 11 und ein computerlesbares Medium gemäß Anspruch 12 gelöst.

Bei dem erfindungsgemäßen Verfahren zur Einstellung eines Kontrasts einer Multi-Energie-CT-Bilddarstellung wird eine Mehrzahl von multispektralen Bilddatensätzen von einem Untersuchungsbereich eines Patienten empfangen. Die multispektralen Bilddatensätze können zum Beispiel im Rahmen eines Bildgebungsverfahrens aktuell erfasst und von einer Rekonstruktionseinheit rekonstruiert worden sein und anschließend direkt mit Hilfe des erfindungsgemäßen Verfahrens weiterverarbeitet werden. Die multispektralen Bilddatensätze können aber auch aus einer Datenbank bzw. einem Datenspeicher stammen, in dem sie längere Zeit zwischengespeichert wurden. Dann wird eine zu untersuchende Struktur auf Basis der multispektralen Bilddatensätze automatisiert identifiziert. Unter einer Identifizierung soll verstanden werden, dass eine Position einer Struktur ermittelt wurde und diese Struktur einer vorbestimmten Klassifizierung zugeordnet wird. Als Struktur soll ein sich von seiner Umgebung unterscheidender Teilbereich in einem Untersuchungsbereich eines Patienten verstanden werden. Schließlich werden die multispektralen Bilddatensätze automatisiert derart unterschiedlich gewichtet kombiniert, dass der Kontrast zwischen der zu untersuchenden Struktur und deren Umgebung im Vergleich zu einer Gleichgewichtung verbessert ist. Vorteilhaft wird die Erkennbarkeit der zu untersuchenden Struktur verbessert, wobei das automatisierte Vorgehen den Aufbereitungsprozess beschleunigt und vereinfacht. Bei einer Mehrzahl von spektralen Bilddatensätzen wird eine optimale Gewichtung der einzelnen Bilddatensätze von Hand sehr aufwändig und aufgrund der Vielzahl an Variationsmöglichkeiten bereits bei einer spektralen Auflösung von mindestens vier Photonenenergien praktisch unmöglich zu bewerkstelligen. Vorteilhaft ermöglicht das erfindungsgemäße Einstellverfahren auch eine schnelle Einstellung von Kontrasten von Multi-Energie-CT-Bilddarstellungen mit einer Vielzahl von unterschiedlichen Röntgenenergiespektren. Da das erfindungsgemäße Verfahren automatisiert abläuft, kann es auch angewendet werden, wenn für die Bildauswertung kein dafür besonders qualifiziertes Personal zur Verfügung steht.

Bei dem erfindungsgemäßen Multi-Energie-CT-Bildgebungsverfahren werden eine Mehrzahl von multi-spektralen Projektionsmessdatensätzen von einem Untersuchungsbereich akquiriert. Nachfolgend wird eine Mehrzahl von multi-spektralen Bilddatensätzen auf Basis der multi-spektralen Projektionsmessdatensätze rekonstruiert. Weiterhin wird eine bezüglich des Kontrasts verbesserte Bilddarstellung auf Basis der rekonstruierten multi-spektralen Bilddatensätze unter Anwendung eines erfindungsgemäßen Verfahrens zur Einstellung eines Kontrasts einer Multi-Energie-CT-Bilddarstellung erzeugt.

Die erfindungsgemäße Bildeinstelleinrichtung weist eine Eingangsschnittstelle zum Empfangen einer Mehrzahl von multi-spektralen Bilddatensätzen von einem Untersuchungsbereich auf. Weiterhin umfasst die erfindungsgemäße Bildeinstelleinrichtung eine Identifizierungseinheit zum automatisierten Identifizieren einer zu untersuchenden Struktur auf Basis der multispektralen Bilddatensätze. Teil der erfindungsgemäßen Bildeinstelleinrichtung ist auch eine Kombinationseinheit zum automatisierten unterschiedlich gewichteten Kombinieren der multispektralen Bilddatensätze derart, dass der Kontrast zwischen der zu untersuchenden Struktur und deren Umgebung im Vergleich zu einer Gleichgewichtung verbessert ist.

Das erfindungsgemäße Computertomographiesystem weist eine Scaneinheit zum Erfassen von Projektionsmessdaten von einem Untersuchungsbereich eines Patienten auf. Teil der erfindungsgemäßen Röntgenbildgebungseinrichtung ist eine Steuerungseinrichtung zum Ansteuern der Scaneinheit und zum Erzeugen von Bilddaten auf Basis der erfassten Projektionsmessdaten. Die erfindungsgemäße Röntgenbildgebungseinrichtung umfasst außerdem eine erfindungsgemäße Bildeinstelleinrichtung.

Die wesentlichen Komponenten der erfindungsgemäßen Bildeinstelleinrichtung können zum überwiegenden Teil in Form von Softwarekomponenten ausgebildet sein. Dies betrifft insbesondere die Identifizierungseinheit und Kombinationseinheit. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher verwendete Computertomographiesysteme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung eines Computertomographiesystems ladbar ist, mit Programmabschnitten, um alle Schritte des erfindungsgemäßen Verfahrens auszuführen, wenn das Computerprogramm in dem Computertomographiesystem ausgeführt wird.

Ein solches Computerprogrammprodukt kann neben dem Computerprogramm gegebenenfalls zusätzliche Bestandteile, wie z.B. eine Dokumentation und/oder zusätzliche Komponenten, auch Hardware-Komponenten, wie z.B. Hardware-Schlüssel (Dongles etc.) zur Nutzung der Software, umfassen

Zum Transport zu einer Speichereinrichtung eines solchen Computertomographiesystems und/oder zur Speicherung an dem Computertomographiesystem kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie oder deren Beschreibungsteilen weitergebildet sein. Zudem können im Rahmen der Erfindung die verschiedenen Merkmale unterschiedlicher Ausführungsbeispiele und Ansprüche auch zu neuen Ausführungsbeispielen kombiniert werden.

In einer Variante des erfindungsgemäßen Verfahrens zur Einstellung eines Kontrasts einer Multi-Energie-CT-Bilddarstellung umfasst die zu untersuchende Struktur eine Läsion.

Wird der Kontrast einer Läsion gegenüber der Umgebung verbessert, so lässt sich die Läsion leichter klassifizieren und damit leichter eine Prognose hinsichtlich ihrer Gefährlichkeit stellen.

Alternativ umfasst die Struktur auch eine anatomische Struktur, wie zum Beispiel ein Organ, eine Gefäßstruktur oder eine Knochenstruktur.

In einer Variante des erfindungsgemäßen Verfahrens zur Einstellung eines Kontrasts einer Multi-Energie-CT-Bilddarstellung erfolgt das Identifizieren der zu untersuchenden Struktur mit Hilfe eines auf maschinellem Lernen basierenden Verfahrens. Ein solches auf dem maschinellen Lernen basierendes Verfahren kann zum Beispiel mit Hilfe einer größeren Anzahl von annotierten Bilddaten trainiert werden, um eine vorbestimmte Struktur, zum Beispiel eine Läsion, im Bild automatisch zu detektieren. Eine solche Trainingsphase erlaubt es einem automatisierten Identifizierungsverfahren, die für eine Identifizierung relevanten Merkmale in Abhängigkeit von der verwendeten Datenbasis für das Training flexibel festzulegen. Mithin ist ein solches Verfahren besonders breit anwendbar, da es sich nicht auf vorab festgelegte starre Regeln oder Modelle stützt.

Dabei erfolgt bei dem erfindungsgemäßen Verfahren zur Einstellung eines Kontrasts einer Multi-Energie-CT-Bilddarstellung das Identifizieren der zu untersuchenden Struktur mit Hilfe eines auf computerassistierter Detektion basierenden Verfahrens. Derartige Verfahren helfen dabei, Bilddaten aus der Computertomographie nach typischen Mustern abzusuchen.

Gemäß einer Alternative des erfindungsgemäßen Verfahrens zur Einstellung eines Kontrasts einer Multi-Energie-CT-Bilddarstellung erfolgt das Ermitteln der unterschiedlichen Gewichtung der Kombination der multispektralen Bilddatensätze auf Basis eines lernenden Systems, welches mit Hilfe von Korrekturen durch den Benutzer trainiert wird. Der dabei eingesetzte Lernprozess wird auch als "deep learning" oder "maschinelles Lernen" bezeichnet. Vorteilhaft kann die Einstellung des Kontrasts vorab durch einen Trainingsprozess an individuelle Anforderungen und Präferenzen eines Benutzers angepasst werden.

Gemäß einer weiteren Alternative des erfindungsgemäßen Verfahrens zur Einstellung eines Kontrasts einer Multi-Energie-CT-Bilddarstellung erfolgt das Ermitteln der unterschiedlichen Gewichtung der Kombination der multispektralen Bilddatensätze regelbasiert. Als regelbasiertes Gewichten soll ein Verfahren verstanden werden, dem zumindest teilweise ein vorab festgelegtes Modell zugrunde liegt. Vorteilhaft ist ein solches System schnell einsetzbar, da es auf festen Regeln basiert und nicht erst trainiert werden muss.

In einer weiteren Variante des erfindungsgemäßen Verfahrens zur Einstellung eines Kontrasts einer Multi-Energie-CT-Bilddarstellung erfolgt bei dem unterschiedlich gewichteten Kombinieren der multispektralen Bilddatensätze eine gewichtete Addition der multispektralen Bilddatensätze. Beispielsweise kann eine Läsion durch eine Injektion vor der Bildgebung mit Iod beaufschlagt worden sein. Dann kann der Kontrast dieser Läsion verstärkt werden, wenn Anteile der Spektraldaten, die möglichst nahe an der K-Absorptionskante von Jod, also bei einer Energie von etwa 33 KeV liegen, stärker gewichtet addiert werden als andere Spektralanteile.

In einer speziellen Variante des erfindungsgemäßen Verfahrens zur Einstellung eines Kontrasts einer Multi-Energie-CT-Bilddarstellung erfolgt bei dem unterschiedlich gewichteten Kombinieren der multispektralen Bilddatensätze eine gewichtete Subtraktion der multispektralen Bilddatensätze. Eine Subtraktion kann zum Beispiel sinnvoll sein, um Hintergrundstrukturen aus einem Bild zu entfernen oder zumindest zu reduzieren. Möchte man zum Beispiel bei einer jodkontrastierten Bilddarstellung nur die Iod-Verteilung darstellen, so können Hintergrundbereiche durch Subtraktion unterschiedlicher Spektralanteile eliminiert werden.

In einer Ausgestaltung des erfindungsgemäßen Verfahrens zur Einstellung eines Kontrasts einer Multi-Energie-CT-Bilddarstellung umfasst das gewichtete Kombinieren der multi-spektralen Bilddatensätze eine nichtlineare Transformation. Ein Beispiel dafür ist, wenn von einem Bilddatensatz nur Daten zu Voxeln verwendet werden, deren Signalintensität über einem definierten Schwellwert liegt. Ein anderes Beispiel liegt vor, wenn alle Voxel, deren Signalintensität in einem ersten Bilddatensatz über einem Schwellwert liegt, ihren Kontrast nur aus einem zweiten Bilddatensatz erhalten, und alle Voxel, deren Intensität darunter liegt, aus einem dritten Bilddatensatz.

In einer Ausgestaltung des erfindungsgemäßen Verfahrens zur Einstellung eines Kontrasts einer Multi-Energie-CT-Bilddarstellung erfolgt das unterschiedlich gewichtete Kombinieren der multispektralen Bilddatensätze derart, dass der Kontrast zwischen der zu untersuchenden Struktur und deren Umgebung optimiert ist. Vorteilhaft wird bei dieser Variante das bestmögliche Kontrastergebnis durch Kombination der multi-spektralen Bilddatensätze erzielt. Hierzu kann zum Beispiel ein automatisiertes Optimierungsverfahren angewandt werden, welches mit Hilfe mathematischer Algorithmen eine Bilddarstellung mit einem optimalen Kontrast, zum Beispiel einem maximal erreichbaren Kontrast zwischen der zu untersuchenden Struktur und deren Umgebung ermittelt.

In einer Variante des erfindungsgemäßen Verfahrens zur Einstellung eines Kontrasts einer Multi-Energie-CT-Bilddarstellung werden die multispektralen Bilddatensätze zusätzlich mit berechneten Bilddaten kombiniert. Ein Beispiel für berechnete Bilddaten ist eine so genannte Jodkarte, bei der der Gehalt an jodhaltigem Kontrastmittel pro Voxel bestimmt wird und in Falschfarben dargestellt wird. Ein weiteres Beispiel ist eine FFR-Berechnung (FFR = fraktionelle Flussreserve), bei der durch eine Strömungssimulation der Strömungswiderstand von Blutgefäßen berechnet wird und ebenfalls in einer Falschfarbendarstellung dargestellt wird. Vorteilhaft können auf diese Weise zusätzliche Informationen in einer einzigen Bilddarstellung angezeigt werden, welche eine Begutachtung zur Stellung einer Diagnose erleichtern.

Die Erfindung wird im Folgenden unter Hinweis auf die beigefügten Figuren anhand von Ausführungsbeispielen noch einmal näher erläutert. Es zeigen:
FIG 1 ein Flussdiagramm, welches ein Verfahren zur Einstellung eines Kontrasts einer Multi-Energie-CT-Bilddarstellung gemäß einem Ausführungsbeispiel der Erfindung veranschaulicht,
FIG 2 eine schematische Darstellung einer Bildeinstelleinrichtung gemäß einem Ausführungsbeispiel der Erfindung,
FIG 3 eine schematische Darstellung eines Computertomographiesystems gemäß einem Ausführungsbeispiel der Erfindung.

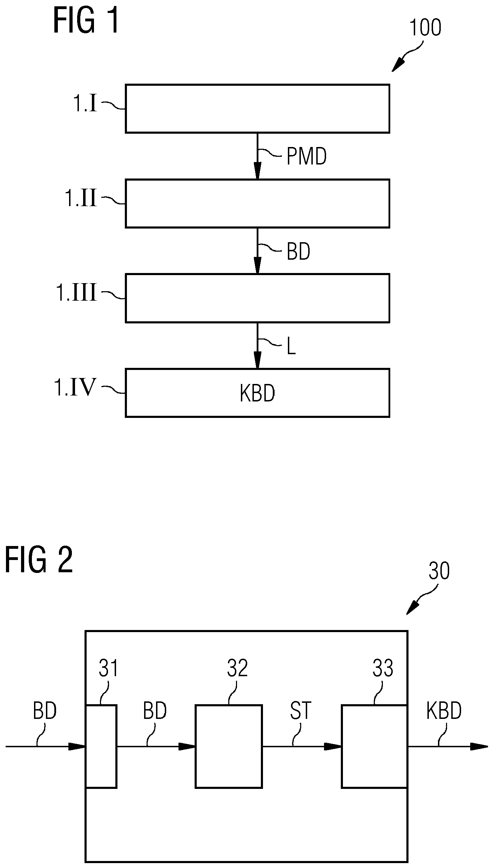

In FIG 1 ist ein Flussdiagramm 100 gezeigt, welches ein Verfahren zur Einstellung eines Kontrasts einer Multi-Energie-CT-Bilddarstellung gemäß einem Ausführungsbeispiel der Erfindung veranschaulicht. Bei dem Schritt 1.I wird zunächst eine Mehrzahl von multi-spektralen Projektionsmessdatensätzen PMD von einem Untersuchungsbereich eines Patienten erfasst. Anschließend werden bei dem Schritt 1.II eine Mehrzahl von multi-spektralen Bilddatensätzen BD auf Basis der multi-spektralen Projektionsmessdatensätze PMD rekonstruiert. Bei dem Schritt 1.III wird eine zu untersuchende Läsion L auf Basis der multispektralen Bilddatensätze BD automatisiert identifiziert. Die Identifizierung kann zum Beispiel mit Hilfe eines Identifizierungsschritts vorgenommen werden, der mit Hilfe eines maschinellen Lernprozesses trainiert wurde, um eine Pathologie, in diesem Fall eine Läsion L, automatisiert zu detektieren. Bei dem Schritt 1.IV werden dann die multi-spektralen Bilddatensätze derart gewichtet, dass der Kontrast zwischen der Läsion L und deren Umgebung im Vergleich zu einer Gleichgewichtung erhöht ist. Beispielsweis kann eine solche Läsion L Jod als Kontrastmittel enthalten. In diesem Fall wird der Kontrast verstärkt, wenn spektrale Anteile, die möglichst nahe an der K-Absorptionskante von Iod liegen, stärker gewichtet werden. Diese Gewichtung kann regelbasiert erfolgen, aber auch durch ein lernendes System, welches anhand von Benutzerrückmeldungen fortlaufend trainiert wird. Bei dem Schritt 1.IV wird also ein kombinierter Bilddatensatz KBD erzeugt, welcher einen optimierten Kontrast zwischen der Läsion L und deren Umgebung aufweist.

In FIG 2 ist eine schematische Darstellung einer Bildeinstelleinrichtung 30 gemäß einem Ausführungsbeispiel der Erfindung gezeigt. Die Bildeinstelleinrichtung 30 umfasst eine Eingangsschnittstelle 31 zum Empfangen einer Mehrzahl von multi-spektralen Bilddatensätzen BD von einem Untersuchungsbereich. Von der Eingangsschnittstelle 31 werden die Bilddaten BD an eine Identifizierungseinheit 32 übermittelt, welche dazu eingerichtet ist, eine zu untersuchende Struktur ST, zum Beispiel eine Läsion L, auf Basis der multispektralen Bilddatensätze BD zu identifizieren. Nach einer Markierung der identifizierten Struktur ST werden die Bilddaten BD an eine Kombinationseinheit 33 übermittelt. Die Kombinationseinheit 33 ist dazu eingerichtet, die multispektralen Bilddatensätze BD derart gewichtet zu kombinieren, dass der Kontrast zwischen der zu untersuchenden Struktur ST und deren Umgebung im Vergleich zu einer Gleichgewichtung verbessert ist.

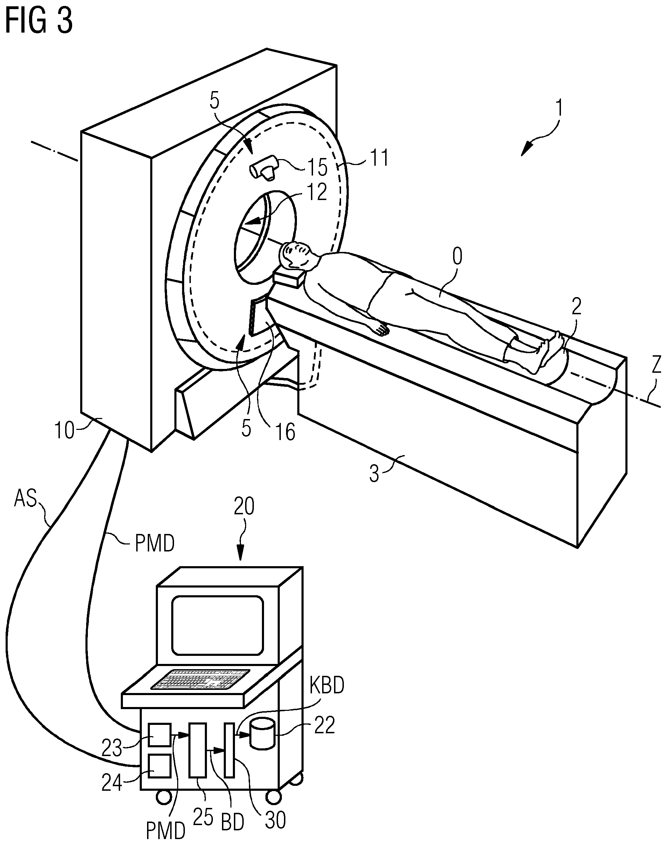

In FIG 3 ist ein Computertomographiesystem 1 gezeigt, welches eine in FIG 2 im Detail gezeigte Bildeinstelleinrichtung 30 umfasst. Das CT-System 1 besteht dabei im Wesentlichen aus einer üblichen Scaneinheit 10, in welcher an einer Gantry 11 eine Projektionsdatenakquisitionseinheit 5 mit einem quantenzählenden Detektor 16 und einer dem quantenzählenden Detektor 16 gegenüberliegenden Röntgenquelle 15 um einen Messraum 12 umläuft. Ein quantenzählender Detektor hat die Fähigkeit, erfasste Röntgenstrahlen hinsichtlich der Energie der Röntgenquanten aufzulösen. Mit einem solchen quantenzählenden Detektor lässt sich die Absorption unterschiedlicher spektraler Anteile der Röntgenstrahlung simultan und nach der Röntgenenergie aufgelöst messen. Vor der Scaneinheit 10 befindet sich eine Patientenlagerungseinrichtung 3 bzw. ein Patiententisch 3, dessen oberer Teil 2 mit einem darauf befindlichen Patienten O zur Scaneinheit 10 verschoben werden kann, um den Patienten O durch den Messraum 12 hindurch relativ zum Detektorsystem 16 zu bewegen. Angesteuert werden die Scaneinheit 10 und der Patiententisch 3 durch eine Steuerungseinrichtung 20, von der aus über eine übliche Steuerschnittstelle 24 Akquisitionssteuersignale AS kommen, um das gesamte System gemäß vorgegebener Messprotokolle in der herkömmlichen Weise anzusteuern. Im Fall einer Spiralakquisition ergibt sich durch eine Bewegung des Patienten O entlang der z-Richtung, welche der Systemachse z längs durch den Messraum 12 entspricht, und den gleichzeitigen Umlauf der Röntgenquelle 15 für die Röntgenquelle 15 relativ zum Patienten O während der Messung eine Helixbahn. Parallel läuft dabei immer gegenüber der Röntgenquelle 15 der Detektor 16 mit, um Projektionsmessdaten PMD zu erfassen, die dann zur Rekonstruktion von Volumen- und/oder Schicht-Bilddaten genutzt werden. Ebenso kann auch ein sequentielles Messverfahren durchgeführt werden, bei dem eine feste Position in z-Richtung angefahren wird und dann während eines Umlaufs, eines Teilumlaufs oder mehrerer Umläufe an der betreffenden z-Position die erforderlichen Projektionsmessdaten PMD erfasst werden, um ein Schnittbild an dieser z-Position zu rekonstruieren oder um aus den Projektionsmessdaten PMD mehrerer z-Positionen Bilddaten BD zu rekonstruieren. Das erfindungsgemäße Verfahren ist grundsätzlich auch an anderen CT-Systemen, z.B. mit mehreren Röntgenquellen und/oder Detektoren und/oder mit einem einen vollständigen Ring bildenden Detektor, einsetzbar. Beispielsweise lässt sich das erfindungsgemäße Verfahren auch auf ein System mit unbewegtem Patiententisch und in z-Richtung bewegter Gantry (einer sogenannten Sliding Gantry) anwenden.

Die vom Detektor 16 akquirierten Projektionsmessdaten PMD (im Folgenden auch Rohdaten genannt) werden über eine Rohdatenschnittstelle 23 an die Steuerungseinrichtung 20 übergeben. Diese Rohdaten PMD werden dann, gegebenenfalls nach einer geeigneten Vorverarbeitung (z. B. Filterung und/oder Strahlaufhärtungskorrektur), in einer Bildrekonstruktionseinrichtung 25 weiterverarbeitet, die in diesem Ausführungsbeispiel in der Steuerungseinrichtung 20 in Form von Software auf einem Prozessor realisiert ist. Diese Bildrekonstruktionseinrichtung 25 rekonstruiert auf Basis der Rohdaten PMD Bilddaten BD mit Hilfe eines Rekonstruktionsverfahrens. Als Rekonstruktionsverfahren kann zum Beispiel ein auf der gefilterten Rückprojektion basierendes Rekonstruktionsverfahren verwendet werden.

Die rekonstruierten Bilddaten BD werden anschließend an eine Bildeinstelleinrichtung 30, wie sie in der FIG 2 im Detail gezeigt ist, übermittelt. Die Bildeinstelleinrichtung 30 erzeugt auf Basis der rekonstruierten Bilddaten BD kombinierte Bilddaten KBD, welche auf die im Zusammenhang mit FIG 1 und 2 beschriebene Art und Weise gewichtet werden.

Die von der Bildeinstelleinrichtung 30 erzeugten kombinierten Bilddaten KBD werden dann in einem Speicher 22 der Steuerungseinrichtung 20 hinterlegt und/oder in üblicher Weise auf dem Bildschirm der Steuerungseinrichtung 20 ausgegeben. Sie können auch über eine in FIG 3 nicht dargestellte Schnittstelle in ein an das Computertomographiesystem 1 angeschlossenes Netz, beispielsweise ein radiologisches Informationssystem (RIS), eingespeist und in einem dort zugänglichen Massenspeicher hinterlegt oder auf dort angeschlossenen Druckern oder Filming-Stationen als Bilder ausgegeben werden. Die Daten können so in beliebiger Weise weiterverarbeitet und dann gespeichert oder ausgegeben werden.

Die Komponenten der Bildeinstelleinrichtung 30 können überwiegend oder vollständig in Form von Softwareelementen auf einem geeigneten Prozessor realisiert sein. Insbesondere können auch die Schnittstellen zwischen diesen Komponenten rein softwaremäßig ausgebildet sein. Erforderlich ist lediglich, dass Zugriffsmöglichkeiten auf geeignete Speicherbereiche bestehen, in denen die Daten geeignet zwischengelagert und jederzeit wieder aufgerufen und aktualisiert werden können.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Verfahren zur Einstellung eines Kontrasts einer Multi-Energie-CT-Bilddarstellung, aufweisend die Schritte:
- Empfangen einer Mehrzahl von multispektralen Bilddatensätzen (BD) von einem Untersuchungsbereich eines Patienten (O),
- automatisiertes Identifizieren einer zu untersuchenden Struktur (ST) auf Basis der multispektralen Bilddatensätze (BD), wobei das Identifizieren der zu untersuchenden Struktur (ST) mit Hilfe eines auf computerunterstützter Detektion basierenden Verfahrens erfolgt und wobei die zu untersuchende Struktur (ST) eine anatomische Struktur oder eine Läsion (L) umfasst,
- automatisiertes, unterschiedlich gewichtetes Kombinieren der multispektralen Bilddatensätze (BD) derart, dass der Kontrast zwischen der zu untersuchenden Struktur (ST) und deren Umgebung im Vergleich zu einer Gleichgewichtung verbessert ist, wobei das Ermitteln der unterschiedlichen Gewichtung der Kombination der multispektralen Bilddatensätze (BD) auf Basis eines lernenden Systems, welches mit Hilfe von Korrekturen durch den Benutzer trainiert wird, oder wobei das Ermitteln der unterschiedlichen Gewichtung der Kombination der multi-spektralen Bilddatensätze (BD) regelbasiert erfolgt.

2. Verfahren nach Anspruch 1, wobei das Identifizieren der zu untersuchenden Struktur (ST) mit Hilfe eines auf maschinellem Lernen basierenden Verfahrens erfolgt.

3. Verfahren nach einem der vorstehenden Ansprüche, wobei bei dem unterschiedlich gewichteten Kombinieren der multispektralen Bilddatensätze (BD) eine gewichtete Addition der multi-spektralen Bilddatensätze (BD) erfolgt.

4. Verfahren nach einem der vorstehenden Ansprüche, wobei bei dem unterschiedlich gewichteten Kombinieren der multispektralen Bilddatensätze (BD) eine gewichtete Subtraktion der multispektralen Bilddatensätze (BD) erfolgt.

5. Verfahren nach einem der vorstehenden Ansprüche, wobei das gewichtete Kombinieren der multispektralen Bilddatensätze (BD) eine nichtlineare Transformation umfasst.

6. Verfahren nach einem der vorstehenden Ansprüche, wobei das unterschiedlich gewichtete Kombinieren der multispektralen Bilddatensätze (BD) derart erfolgt, dass der Kontrast zwischen der zu untersuchenden Struktur (ST) und deren Umgebung optimiert ist.

7. Verfahren nach einem der vorstehenden Ansprüche, wobei die multispektralen Bilddatensätze (BD) zusätzlich mit berechneten Bilddaten (BD) kombiniert werden.

8. Multi-Energie-CT-Bildgebungsverfahren, aufweisend die Schritte:
- Erfassen einer Mehrzahl von multi-spektralen Projektionsmessdatensätzen (PMD) von einem Untersuchungsbereich eines Patienten (O),
- Rekonstruieren einer Mehrzahl von multi-spektralen Bilddatensätzen (BD) auf Basis der multi-spektralen Projektionsmessdatensätze (PMD),
- Erzeugen einer bezüglich des Kontrasts verbesserten Bilddarstellung (KBD) auf Basis der rekonstruierten multi-spektralen Bilddatensätze (BD) unter Anwendung eines Verfahrens nach einem der Ansprüche 1 bis 7.

9. Bildeinstelleinrichtung (30), aufweisend:
- eine Eingangsschnittstelle (31) zum Empfangen einer Mehrzahl von multispektralen Bilddatensätzen (BD) von einem Untersuchungsbereich eines Patienten (O),
- eine Identifizierungseinheit (32) zum automatisierten Identifizieren einer zu untersuchenden Struktur (ST) auf Basis der multispektralen Bilddatensätze (BD), die ausgelegt ist, die zu untersuchende Struktur (ST) mit Hilfe eines auf computerunterstützter Detektion basierenden Verfahrens zu identifizieren, wobei die zu untersuchende Struktur (ST) eine anatomische Struktur oder eine Läsion (L) umfasst,
- eine Kombinationseinheit (33) zum automatisierten unterschiedlich gewichteten Kombinieren der multispektralen Bilddatensätze (BD) derart, dass der Kontrast zwischen der zu untersuchenden Struktur (ST) und deren Umgebung im Vergleich zu einer Gleichgewichtung verbessert ist, wobei die Kombinationseinheit derart ausgelegt ist, dass das Ermitteln der unterschiedlichen Gewichtung der Kombination der multispektralen Bilddatensätze (BD) auf Basis eines lernenden Systems, welches mit Hilfe von Korrekturen durch den Benutzer trainiert ist, oder derart ausgelegt ist, dass das Ermitteln der unterschiedlichen Gewichtung der Kombination der multispektralen Bilddatensätze (BD) regelbasiert erfolgt.

10. Computertomographiesystem (1), aufweisend:
- eine Scaneinheit (10) zum Erfassen von Projektionsmessdaten (PMD) von einem Untersuchungsbereich eines Patienten (P),
- eine Steuerungseinrichtung (20) zum Ansteuern der Scaneinheit (31) und zum Erzeugen von Bilddaten (BD) auf Basis der erfassten Projektionsmessdaten (PMD),
- eine Bildeinstelleinrichtung (30) nach Anspruch 9.

11. Computerprogrammprodukt mit einem Computerprogramm, welches direkt in eine Speichereinheit eines Computertomographiesystems (1) ladbar ist, mit Programmabschnitten, um alle Schritte eines Verfahrens nach einem der Ansprüche 1 bis 8 auszuführen, wenn das Computerprogramm in dem Computertomographiesystem (1) ausgeführt wird.

12. Computerlesbares Medium, auf welchem von einer Recheneinheit ausführbare Programmabschnitte gespeichert sind, um alle Schritte des Verfahrens nach einem der Ansprüche 1 bis 8 auszuführen, wenn die Programmabschnitte von der Recheneinheit ausgeführt werden.

## Claims

1. Method for setting a contrast of a multi-energy CT image representation, having the steps of:
- receiving a plurality of multispectral image datasets (BD) of an examination region of a patient (O),
- automatically identifying a structure (ST) to be examined on the basis of the multispectral image datasets (BD), wherein the structure (ST) to be examined is identified with the aid of a method based on computer-assisted detection and wherein the structure (ST) to be examined comprises an anatomical structure or a lesion (L),
- automatically combining the differently weighted multispectral image datasets (BD) in such a way that the contrast between the structure (ST) to be examined and its environment is improved compared to a uniform weighting, wherein the different weighting of the combination of multispectral image datasets (BD) is determined on the basis of a learning system which is trained with the aid of corrections by the user, or wherein the different weighting of the combination of multispectral image datasets (BD) is determined using a rule-based approach.

2. Method according to claim 1, wherein the structure (ST) to be examined is identified with the aid of a method based on machine learning.

3. Method according to one of the preceding claims, wherein the differently weighted combining of the multispectral image datasets (BD) includes a weighted addition of the multispectral image datasets (BD).

4. Method according to one of the preceding claims, wherein the differently weighted combining of the multispectral image datasets (BD) includes a weighted subtraction of the multispectral image datasets (BD).

5. Method according to one of the preceding claims, wherein the weighted combining of the multispectral image datasets (BD) comprises a nonlinear transformation.

6. Method according to one of the preceding claims, wherein the differently weighted combining of the multispectral image datasets (BD) is accomplished in such a way that the contrast between the structure (ST) to be examined and its environment is optimized.

7. Method according to one of the preceding claims, wherein the multispectral image datasets (BD) are additionally combined with calculated image data (BD).

8. Multi-energy CT imaging method, having the steps of:
- acquiring a plurality of multispectral projection measurement datasets (PMD) of an examination region of a patient (O),
- reconstructing a plurality of multispectral image datasets (BD) on the basis of the multispectral projection measurement datasets (PMD),
- generating an image representation (KBD) that is improved in terms of contrast on the basis of the reconstructed multispectral image datasets (BD) using a method according to one of claims 1 to 7.

9. Image setting device (30) having:
- an input interface (31) for receiving a plurality of multispectral image datasets (BD) of an examination region of a patient (O),
- an identification unit (32) enabling the automated identification of a structure (ST) to be examined on the basis of the multispectral image datasets (BD), which is designed to identify the structure (ST) to be examined with the aid of a method based on computer-assisted detection, wherein the structure (ST) to be examined comprises an anatomical structure or a lesion (L),
- a combination unit (33) enabling the automated combining of the differently weighted multispectral image datasets (BD) in such a way that the contrast between the structure (ST) to be examined and its environment is improved compared to a uniform weighting, wherein the combination unit is designed in such a way that the different weighting of the combination of multispectral image datasets (BD) is determined on the basis of a learning system which is trained with the aid of corrections by the user, or is designed in such a way that the different weighting of the combination of multispectral image datasets (BD) is determined using a rule-based approach.

10. Computed tomography system (1) having:
- a scan unit (10) for acquiring projection measurement data (PMD) of an examination region of a patient (P),
- a control device (20) for controlling the scan unit (31) and for generating image data (BD) on the basis of the acquired projection measurement data (PMD),
- an image setting device (30) according to claim 9.

11. Computer program product having a computer program which can be loaded directly into a memory unit of a computed tomography system (1), and having program sections for the purpose of performing all steps of a method according to one of claims 1 to 8 when the computer program is executed in the computed tomography system (1).

12. Computer-readable medium on which are stored program sections that are executable by a computer unit for the purpose of performing all steps of the method according to one of claims 1 to 8 when the program sections are executed by the computer unit.

## Revendications

1. Procédé de réglage d'un contraste d'une représentation d'image par tomodensitométrie assistée par ordinateur à multi-énergies, comprenant les stades :
- réception d'une pluralité d'ensembles (BD) de données d'image multispectraux d'une partie à examiner d'un patient (O),
- identification automatisée d'une structure (ST) à examiner sur la base des ensembles (BD) de données d'image multispectraux, dans lequel l'identification de la structure (ST) à examiner s'effectue à l'aide d'un procédé reposant sur une détection assistée par ordinateur et dans lequel la structure (ST) à examiner comprend une structure anatomique ou une lésion (L),
- combinaison automatisée et pondérée différemment des ensembles (BD) de données d'image multispectraux, de manière à améliorer le contraste entre la structure (ST) à examiner et son environnement par rapport à une équipondération, dans lequel la détermination de la pondération différente de la combinaison des ensembles (BD) de données d'image multispectraux s'effectue sur la base d'un système d'enseignement, qui subit un apprentissage par l'utilisateur à l'aide de corrections, ou dans lequel la détermination de la pondération différente de la combinaison des ensembles (BD) de données d'image multispectraux s'effectue sur la base d'une règle.

2. Procédé suivant la revendication 1, dans lequel l'identification de la structure (ST) à examiner s'effectue à l'aide d'un procédé reposant sur un enseignement par machine.

3. Procédé suivant l'une des revendications précédentes, dans lequel, lors de la combinaison pondérée différemment des ensembles (BD) de données d'image multispectraux, une addition pondérée des ensembles (BD) de données d'image multispectraux a lieu.

4. Procédé suivant l'une des revendications précédentes, dans lequel, lors de la combinaison pondérée différemment des ensembles (BD) de données d'image multispectraux, une soustraction pondérée des ensembles (BD) de données d'image multispectraux a lieu.

5. Procédé suivant l'une des revendications précédentes, dans lequel, la combinaison pondérée des ensembles (BD) de données d'image multispectraux comprend une transformation non linéaire.

6. Procédé suivant l'une des revendications précédentes, dans lequel, la combinaison pondérée différemment des ensembles (BD) de données d'image multispectraux s'effectue de manière à optimiser le contraste entre la structure (ST) à examiner et ce qui l'entoure.

7. Procédé suivant l'une des revendications précédentes, dans lequel, on combine les ensembles (BD) de données d'image multispectraux supplémentairement à des données (BD) d'image calculées.

8. Procédé d'imagerie par tomodensitométrie assistée par ordinateur à multi-énergies, comportant les stades :
- détection d'une pluralité d'ensembles (PMD) de données de mesure de projection multispectraux d'une partie à examiner d'un patient (O),
- reconstruction d'une pluralité d'ensembles (BD) de données d'image multispectraux sur la base des ensembles (PMD) de données de mesure de projection multispectraux,
- production d'une représentation (KBD) d'image améliorée en ce qui concerne le contraste sur la base des ensembles (BD) de données d'image multispectraux reconstruits en appliquant un procédé suivant l'une des revendications 1 à 7.

9. Dispositif (30) de réglage d'image, comportant :
- une interface (31) d'entrée pour la réception d'une pluralité d'ensembles (BD) de données d'image multispectraux d'une partie à examiner d'un patient (O),
- une unité (32) d'identification pour l'identification automatisée d'une structure (ST) à examiner sur la base des ensembles (BD) de données d'image multispectraux, qui est conçue pour identifier la structure (ST) à examiner à l'aide d'un procédé reposant sur une détection assistée par ordinateur, dans lequel la structure (ST) à examiner comprend une structure anatomique ou une lésion (L),
- une unité (33) de combinaison pour la combinaison automatisée et/ou pondérée différemment des ensembles (BD) de données d'image multispectraux, de manière à améliorer le contraste entre la structure (ST) à examiner et ce qui l'entoure, par rapport à une équipondération, dans lequel l'unité de combinaison est conçue de manière à ce que la détermination de la pondération différente de la combinaison des ensembles (BD) de données d'image multispectraux s'effectue sur la base d'un système d'enseignement, qui subit un apprentissage par l'utilisateur à l'aide de corrections, ou qui est conçu de manière à ce que la détermination de la pondération différente de la combinaison des ensembles (BD) de données d'image multispectraux s'effectue sur la base d'une règle.

10. Système (1) de tomodensitométrie assisté par ordinateur, comportant :
- une unité (10) de scan pour la détection de données (PMD) de mesure de projection d'une partie à examiner d'un patient (P),
- un dispositif (20) de commande pour la commande de l'unité (31) de scan et pour la production de données (BD) d'image sur la base des données (PMD) de mesures de projections détectées,
- un dispositif (30) de réglage d'image suivant la revendication 9.

11. Produit de programme d'ordinateur, comprenant un programme d'ordinateur, qui peut être chargé directement dans une unité de mémoire d'un système (1) de tomodensitométrie assisté par ordinateur, comprenant des parties de programme pour effectuer tous les stades d'un procédé suivant l'une des revendications 1 à 8, lorsque le programme d'ordinateur est réalisé dans le système (1) de tomodensitométrie assistée par ordinateur.

12. Support, déchiffrable par ordinateur, sur lequel sont mémorisées des parties de programme réalisables par une unité informatique, afin d'effectuer tous les stades du procédé suivant l'une des revendications 1 à 8, lorsque les parties de programme sont réalisées par l'unité informatique.
